# EUROPÄISCHE PATENTSCHRIFT

(11) **EP 0 574 480 B1**
(45) Veröffentlichungstag und Bekanntmachung des Hinweises auf die Patenterteilung: **10.01.1996**
(21) Anmeldenummer: 92906267.7
(22) Anmeldetag: 05.03.1992
(51) Int. Cl.: A61B 5/20, G01F 3/38, G01W 1/14

(54) **VORRICHTUNG ZUR MESSUNG DES VOLUMENS EINES FLÜSSIGKEITSSTRÖMES**
DEVICE FOR MEASURING THE VOLUME OF A FLOWING LIQUID
DISPOSITIF POUR LA MESURE DU VOLUME D'UN FLUX DE LIQUIDE

(30) Priorität: 05.03.1991 DE 4106995
(43) Veröffentlichungstag der Anmeldung: 22.12.1993
(73) Patentinhaber: DURANGO HOLDING GMBH, D-85049 Ingolstadt (DE)
(72) Erfinder: DIETZ, Xaver, D-8075 Vohburg (DE); KOTHMEIER, Georg, D-8070 Ingolstadt (DE)
(74) Vertreter: Heim, Hans-Karl, Dipl.-Ing.
(86) Internationale Anmeldenummer: EP9200499
(87) Internationale Veröffentlichungsnummer: WO9215249

(56) Entgegenhaltungen:
- DE-A- 3 500 895
- DE-A- 3 544 676
- GB-A- 2 031 158
- US-A- 4 589 280
- US-A- 4 619 273

## Beschreibung

Die Erfindung betrifft eine Vorrichtung zur Messung eines Flüssigkeitsstromes gemäß Oberbegriff des Anspruchs 1.

Im Laborbereich und insbesondere im medizinischen Bereich besteht Bedarf nach einer Möglichkeit, geringe Volumenströme von Flüssigkeiten mit hoher Genauigkeit zu messen. Dabei treten vor allem im klinischen Bereich einige Probleme auf.

Die zu messenden Flüssigkeiten sind bisweilen chemisch-aggressive Körperflüssigkeiten, die unter anderem mit Feststoffen, zum Beispiel Eiweiß durchsetzt sind. Für die Messung derartiger Flüssigkeiten ist ein einfacher Aufbau der Meßapparatur notwendig, der Verunreinigungen möglichst wenig Angriffsfläche bietet. Dieser einfache Aufbau kommt weiterhin den hohen Hygieneanforderungen eines klinischen Betriebs entgegen.

Grundsätzlich sind verschiedene Prinzipien zur Erfassung des Volumens eines Flüssigkeitsstromes, insbesondere bei Urimetern, bekannt.

Ein gattungsgemäßes Verfahren und eine gattungsgemäße Vorrichtung der eingangs genannten Art sind aus der US-PS 4,619,273 bekannt.

Das hieraus bekannte Verfahren und die Vorrichtung sind primär darauf ausgerichtet, die Abgabegeschwindigkeit bei einem urinabgebenden Patienten zu erfassen und über eine nachgeordnete Siphon-Anordnung auch die quantifizierte Menge der abgegebenen Flüssigkeit festzustellen.

Dieses Verfahren und die Vorrichtung sind daher auf eine relativ großvolumige spontane Abgabe einer Flüssigkeit ausgelegt. Durch diese hohe Durchflußrate wird über den ringförmig um den Befüllungskanal vorgesehenen und mit der Umgebung in Verbindung stehenden Belüftungskanal entsprechend der durchströmenden Flüssigkeit Luft eingesaugt, wodurch Luftblasen zur Separierung einzelner Flüssigkeitsvolumen voneinander erzeugt werden. Wollte man diese Vorrichtung zur Erfassung des Volumens sehr kleiner abgegebener Volumina, wie sie bei Patienten auftreten, die einen Blasenkatheter gelegt bekommen haben, einsetzen, so würde aufgrund der geometrischen Auslegung der Siphon-Anordnung, in der die unterste Rundung des Siphon-Ablaufsbereichs, welcher in einer horizontalen Ebene der oberen Rundung des Einlaufsbereichs liegt, bei Erreichen dieses horizontalen Niveaus ein Ablaufen der Flüssigkeit in Art einer Rieselfilmbildung eintreten. Dies gestattet jedoch keine definierte Erfassung des abströmenden Volumens und erst recht nicht die Bildung gleich großer Meßvolumina.

In dieser Druckschrift wird daher darauf abgestellt, daß die beschriebene Vorrichtung zum Messen hoher Flüssigkeitsströme geeignet, ist und nur dort das Meßverfahren funktioniert. Es wird hierbei unter der Wirkung eines Flüssigkeitspfropfens im Auslaufrohr die entsprechende Flüssigkeitsmenge aus dem Siphon gesogen.

Bei einem langsamen Flüssigkeitsfluß würde dieser Pfropfen sich der Wandung des Siphons entlang abrieselnd auflösen, so daß die Sogwirkung und damit auch die Funktion der Meßerfassung verlorengeht.

Weiterhin sind Meßvorrichtungen bekannt (US-PS 4,683,748 und 4,589,280), die zur Aufnahme eines größeren Meßvolumens abgegebenen Urins ausgelegt ist. Von der entsprechenden Meßkammer verläuft vom Boden ausgehend nach oben ein Druckkanal, der durch einen die Analogwerte des Druckanstieges erfassenden Transducer abgeschlossen ist. Diese analogen Druckwerte können dann über eine elektronische Auswertschaltung in die Füllhöhe der Meßkammer und damit das Meßvolumen umgerechnet werden.

Um einen ungewollten Rückstau des abgegebenen Urins zu vermeiden und Kontaminationen auszuschließen, weist diese Meßvorrichtung auch einen Überlauf in Art eines Siphon auf, dessen Einlaßkanal erheblich über dem untersten Niveau der Meßkammer beginnt. Die Funktion dieser auch als "Saugheber-Prinzip" bezeichneten Siphon-Anordnung kann daher als reine Überlauffunktion angesehen werden, die über eine Teilentleerung der Meßkammer einen entsprechenden Druckunterschied für den Transducer hervorrufen soll.

Problematisch ist bei dieser Meßvorrichtung der komplizierte Aufbau der elektronischen Auswerteinrichtung, wobei bereits die Analogerfassung des indirekten Parameters des Druckes erhebliche Fehler hervorrufen kann. Zum anderen werden Sedimente, die mit dem Urin ausgeschwemmt werden, gerade in der Meßkammer abgelagert, ohne daß diese Sedimente auch ausgespült werden, so daß auch hierdurch eine Ungenauigkeit des Meßergebnisses hervorgerufen wird.

Bei einer anderen Meßvorrichtung (DE 35 44 676 A1) werden Parameter des abgegebenen Flüssigkeitsstromes, wie spezifisches Gewicht, Temperatur etc., über eine relativ komplizierte Meßkammeranordnung mit nachgeschalteter elektronischer Auswerteinrichtung berechnet. Diese Meßvorrichtung ist primär für eine kontinuierliche Erfassung qualitativer Parameter des Urin-Flüssigkeitsstromes bestimmt. Eine Siphon-Anordnung, die auch hier aufgegriffen ist, dient einzig und allein zur Bestimmung der spezifischen Parameter in voneinander separierten, einzelnen Flüssigkeitsvolumen, ohne daß eine Vermischung vor sich gehen soll. Das Volumen des Flüssigkeitsstromes selbst wird jedoch im wesentlichen über eine Gewichtsmessung der durchlaufenden Flüssigkeitsströmung mittels Dehnungsmeßstreifen durchgeführt.

Eine derartige Meßvorrichtung ist daher gerade aufgrund ihrer Komplexität erheblich fehlerbehaftet, was insbesondere auch für die Gewichts- und damit abgegebene Volumenbestimmung der Flüssigkeitsströmung zutrifft.

Weiterhin ist aus der US-PS 3,919,455 (Fig. 1) eine Meßvorrichtung bekannt, in deren zylinderförmigen Behälter ein mit der Öffnung nach unten weisendes U-Rohr hineinragt. Dieser Behälter wird ohne erkennbare Be- und Entlüftungsleitungen mit Flüssigkeit gefüllt, bis das oberste Niveau des U-förmigen Siphons überschritten ist. Zu diesem Zeitpunkt setzt dann die Saugheber-Funktion ein und das relativ große Volumen aus dem Behälter wird bis auf das unterste Niveau des U-Rohres entleert. Das Fehlen einer Be- und Entlüftung kann dazu führen, daß bei vollständigem Ausfüllen des Befüllungskanals durch die zugeführte Flüssigkeit ein ansteigender Luftdruck im Behälter entsteht, der die Saugheber-Funktion weit vor dem Erreichen des obersten Flüssigkeitsniveaus auslöst, so daß unterschiedliche Meßvolumina im Ablaufkanal digital erfaßt werden.

In gleicher Weise arbeitet auch die aus der GB 20 31 158 A1 bekannte Meßvorrichtung.

Als nachteilig kann bei den beiden zuletzt genannten Meßvorrichtungen angesehen werden, daß keine vollständige Entleerung der Meßkammer durchgeführt wird und stets aufgrund des Abstandes zwischen der unteren Ansaugöffnung und dem Boden der Meßkammer ein Restvolumen in der Meßkammer verbleibt. Je kleiner jedoch dieser Abstand gewählt wird, umso mehr wird der Abfluß der Flüssigkeit durch das Saugrohr gebremst, da die Strömungsgeschwindigkeit durch den schmaler werdenden Spalt beeinträchtigt wird.

Hiermit verbunden sind jedoch weitere Nachteile. Zum einen führt die geringer werdende Abflußgeschwindigkeit auch zu einer Meßungenauigkeit, da während des digital erfaßten, relativ langsamen Ablaufens Flüssigkeit durch das Saugrohr bereits wieder Flüssigkeit von oben im Befüllungskanal nachströmt, die daher mit in das Meßvolumina eingeht. Eine für die digitale Erfassung wichtige exakte Proportionierung wird daher verwischt.

Ein weiterer Nachteil liegt in der verminderten Fähigkeit dieser Saugheber, Sedimente, die sich in der Flüssigkeit befinden oder die sich am Boden der Meßkammer abgesetzt haben, mitzureißen.

Weiterhin treten an den unteren Kanten des Saugheberrohres Verwirbelungen auf, die eine optimale Strömungsdynamik beeinträchtigen.

Hinzu treten die relativ großen Meßvolumina, die in diesen Meßkammer vorhanden sind, wobei allein durch die benetzten großen Oberflächen unterschiedliche Strömungscharakteristiken und damit Fehler auftreten.

Auch kann als nachteilig angesehen werden, daß bei leichter Schräglage der bekannten Meßanordnungen Luft zum Ende des Absaugvorgangs mitabgesaugt wird, was zu einem Luft-/Flüssigkeitsgemisch führt und Fehlzählungen bei der digitalen elektronischen Meßvorrichtung auslösen kann, da ein eindeutiger, abschließender Flüssigkeitsmeniskus nicht vorhanden ist.

Problematisch bei diesen bekannten Meßvorrichtungen, insbesondere mit großer Benetzungsoberfläche in der Meßkammer, ist auch, daß das Benetzungsverhalten von Flüssigkeiten auf Oberflächen eine mathematisch nicht exakt beschreibbare Hysterese aufweist, sich also stochastisch verhält. Hierbei ist insbesondere die Adhäsionskraft der Flüssigkeit an der Wandung von großem Einfluß und daher im voraus nicht berechenbar. Es treten daher bei derartigen Meßvorrichtungen große Varianzen der einzelnen Meßvolumina auf, die selbstverständlich die Genauigkeit der digitalen Erfassung negativ beeinflussen.

Insgesamt gesehen weisen die bekannten Meßvorrichtungen hohe Ungenauigkeiten im Hinblick auf die einzelnen Meßvolumina auf. Die Vorrichtungen sind üblicherweise relativ komplex und damit mit vergleichsweise hohen Kosten verbunden. Aufgrund großer Oberflächen in den Meßkammern ergeben sich zusätzliche Probleme, die auch hohe Neigungsempfindlichkeit mit sich bringen.

Es ist daher **Aufgabe** der Erfindung, ein Verfahren und eine relativ einfach und kostengünstig konzipierte Vorrichtung zur Messung des Volumens eines Flüssigkeitsstroms zu schaffen, die insbesondere auch im klinischen Bereich, z.B. auf Intensivstationen, zur Bilanzierung der Urinausscheidung bei katheterisierten Patienten, einsetzbar ist und die eine funktionssichere und sehr genaue Messung des Flüssigkeitsstroms ermöglicht.

Diese Aufgabe wird erfindungsgemäß durch eine Vorrichtung gemäß Anspruch 1 gelöst.

Ein wesentlicher Grundgedanke der Erfindung kann in der geometrischen Auslegung der Siphon-Anordnung gesehen werden, die es erlaubt, eine Quantifizierung des zugelaufenen Flüssigkeitsstroms in weitestgehend identische Meßvolumina durchzuführen und allein das Vorhandensein bzw. das Ablaufen und Entleeren des Meßvolumens mittels einer Sprungantwort der Detektoranordnung, die dementsprechend impulsartig und digital erfolgt, festzustellen. Auf diese Weise kann mittels des sehr kleinen, definierten, einzelnen Meßvolumens und der Anzahl der durchgelaufenen Meßvolumina das Gesamtvolumen des Flüssigkeitsstroms zeitabhängig präzise ausgewertet werden.

Die Erfindung geht daher von dem Grundprinzip aus, Siphon und Kammer dicht benachbart zueinander vorzusehen und diese nach Möglichkeit als Rohr oder Rohrerweiterung auszubilden, um als einzelne Meßvolumina möglichst kleine identische Volumina zu erhalten.

Der kontinuierliche Übergang zwischen Kammer und Siphon, wobei zweckmäßigerweise eine Bodenabschrägung zum Siphon hin vorgesehen sein sollte, ermöglichen optimale Strömungsverhältnisse, so daß die Entleerung schnellstmöglich vor sich geht.

Hierbei wird die lichte Weite des Siphons und der Meßkammer, insbesondere für Urinmessung, auf einen Durchmesser von minimal etwa 4 mm ausgelegt, so daß hierbei Sedimente noch beim Abfließen des einzelnen Meßvolumina mitgerissen werden können. Der Durchmesser des Siphons muß andererseits im Hinblick auf die Flüssigkeit und deren Viskosität so klein ausgelegt sein, daß sich ein eindeutiger Flüssigkeitsmeniskus bilden kann. Hierbei ist selbstverständlich auch die Materialhydrophobie mitzuberücksichtigen und der Aspekt, daß keine Kapillarwirkungen auftreten dürfen.

Da bei der Erfindung somit Kanten und scharfe Krümmungsradien vermieden werden und ein annähernd gleichbleibender Strömungsquerschnitt in Meßkammer und Siphon vorhanden ist, kann am Ende eines jeden Meßzyklus ein genau definierter Flüssigkeitsmeniskus gebildet werden, der auch eine exakte digitale Zählung zuläßt. Unabhängig von der Neigung der Anordnung um eine Achse, die senkrecht zu deren Symmetrieebene liegt, wird immer ein deutlicher Endmeniskus gebildet.

Um eine weitere Genauigkeitsverbesserung bei den Meßvolumina herbeizuführen, werden vorteilhafterweise Beeinflussungen über die Neigung der Siphon-Anordnung und die Durchflußmenge mittels der elektronischen Detektoreinrichtung eliminiert.

Das erfindungsgemäße Konzept kann dahingehend umrissen werden, daß vom Boden der Meßkammer aus ein mit dem Siphon verbundenes Rohr rüsselartig zum Siphon führt und in diesen übergeht. Der Querschnitt dieses Rohres entspricht etwa demjenigen des Siphons. Hierbei ist weiterhin ein Gefälle vom Boden der Kammer zum Einlauf des Siphons vorgesehen. Andererseits wird der Zulauf zur Kammer durch ein Befüllungsrohr relativ kleinen Durchmessers gebildet, so daß auch bei einer Schrägstellung und Neigung der Siphon-Anordnung ein nur sehr geringer Volumenfehler auftreten kann, der detektormäßig eliminierbar ist.

Die erfindungsgemäße Meßvorrichtung zeichnet sich weiterhin dadurch aus, daß der Siphon außenliegend zur Kammer vorgesehen ist und der Entleerungsvorgang der insbesondere als Rohrerweiterung ausgebildeten Kammer von unten her erfolgt. Hierbei sind homogene Krümmungen in Kammer und Siphon vorgesehen, damit die Strömungsdynamik störende Kanten vermieden werden. Die Kammer wird hierbei zweckmäßigerweise als Rohrerweiterung ausgelegt, wobei diese z.B. kegel- bzw. kugelförmig oder als ovaler Rotationsellipsoid ausgebildet sein kann.

Der Befüllungskanal zur Kammer hin ist vorzugsweise als Schrägkanal ausgebildet, wobei auch ein kleinerer Durchmesser als bei der Kammer vorgesehen sein kann.

Zur günstigen Beeinflussung der Ablaufcharakteristik ist der Abflußkanal schräg angeschnitten in den Rückschlußkanal eingeführt, wobei auch eine trompetenförmige Erweiterung vorgesehen sein kann.

Zur eindeutigen Bildung eines Flüssigkeitsmeniskus kann hinter dem obersten Niveau des Siphons ein Durchmessersprung vorhanden sein. In vergleichbarer Weise kann auch ein Durchmessersprung am Übergang vom Befüllungskanal zur Kammer ausgebildet sein.

Vorteilhafterweise wird eine interne Be- und Entlüftung gewählt, die vom Bereich des Ablaufkanals über das höchste Niveau des Siphons vor oder nach der Kammer zugeführt ist.

Im Hinblick auf eine optimale Überwachung wird die Siphon-Anordnung aus einem durchsichtigen Kunststoff hergestellt. Dies kann zweckmäßigerweise aus zwei weitgehend identischen Halbblöcken geschehen, die gespritzt sind, und z.B. mittels eines Ultraschweißverfahrens oder Klebens bzw. Schraubens miteinander dicht verbunden werden können. Das entsprechende Material ist geeigneterweise hydrophob. Die gesamte Siphon-Anordnung kann daher im Sinne eines Einwegartikels hergestellt werden. Im Hinblick auf eine höhere Umweltfreundlichkeit kann auch ein biologisch abbaubarer bzw. verrottbarer Kunststoff, z. B. Biocellat (WZ, Battelle-Institut, Frankfurt a.M.), verwendet werden, wobei ein Cellulosediacetat als Grundsubstanz in Frage kommt, das mit Weichmachern versetzt ist. Andere Kunststoffe auf Pflanzenölbasis können ebenfalls spritztechnisch verwendet werden.

In einer weiter vereinfachten Form wird innerhalb von Führungszapfen der Kunststoff-Halbblöcke ein durchsichtiger Kunststoffschlauch eingelegt, der Zulaufkanal, Kammer, Siphon und Ablaufkanal bildet. Vor dem Zu- und nach dem Ablauf werden in diesem Fall Filter eingesetzt, die das Innere des Schlauches belüften.

Der Vorteil dieser Ausführungsform besteht darin, daß allein der Schlauch nun ausgetauscht und als Einwegartikel angesehen werden kann, während die beiden Kunststoff-Blockhälften wiederverwendbar sind.

Die elektronische Detektoranordnung weist vorzugsweise Korrelationseinrichtungen zur Eliminierung von Meßfehlern infolge von Neigungen der Siphon-Anordnung und aufgrund unterschiedlicher Durchflußvolumina auf.

Die Auslegung der Siphon-Anordnung selbst, gekoppelt mit dem Schritt der einfachen digitalen Erfassung des Abfließens beziehungsweise Vorhandenseins des einzelnen Meßvolumens, erlaubt daher eine kostengünstige, präzis arbeitende Meßvorrichtung, die z.B. durch die indirekte optische Erfassung der einzelnen Meßvolumina in Industrie, Chemie, Umwelttechnik oder Klinik unabhängig von der Aggressivität oder Kontaminationsmöglichkeit des Flüssigkeitsstromes einsetzbar ist.

Durch das vorzugsweise Ausrichten einer optischen Reflex-Lichtschranke in eine im Aufstaubereich der Siphon-Anordnung befindliche Kammer wird die Anzahl der Entleerungs- oder Siphon-Zyklen präzis erfaßt. Eine Messung im Ablaufbereich der Siphon-Anordnung ist ebenfalls möglich.

Um eine sehr genaue Volumenstrommessung durchführen zu können, ist es erwünscht, daß das Volumen in der Kammer der Siphon-Anordnung genau definiert ist. Eine exakte Festlegung des Meßvolumens in der Kammer läßt sich erreichen, wenn der Siphon im Bereich der Überlaufkante einen Durchmessersprung aufweist. Beim Aufstauen der Flüssigkeit in der Meßkammer bildet sich an diesem Durchmessersprung aufgrund der Oberflächenspannung der Flüssigkeit eine Membran. Eine Entleerung der Kammer erfolgt dann, wenn die Höhe der aufgestauten Flüssigkeit in der Meßkammer die Oberflächenspannung dieser Membran überschreitet. Dies ist ein definierter Auslösepunkt, verglichen mit herkömmlichen Siphon-Anordnungen. Dort staut sich die Flüssigkeit je nach Oberflächenspannung bis über die Überlaufkante, wobei jedoch eine vorzeitige Auslösung durch Turbulenzen aufgrund zulaufender Flüssigkeit oder aufgrund geringfügiger Lageveränderungen der Siphon-Anordnung erfolgen kann.

Die für die Auslösung der Entleerung und die Entleerung maßgeblichen Strömungsverhältnisse der Flüssigkeit in der Siphon-Anordnung lassen sich abermals verbessern, wenn die Siphon-Anordnung aus einem hydrophoben Material besteht und/oder hydrophob behandelt ist.

Um eine geringe Abhängigkeit der Meßvolumen von der Lage der Siphon-Anordnung zu erhalten, ist es weiterhin günstig ein von der Meßkammer nach oben weisendes Steigrohr mit einem kleinen Durchmesser vorzusehen, weil die durch eine Verdrehung oder Kippung der Siphon-Anordnung erfolgende frühere oder spätere Auslösung des Entleerungsvorgangs zu kleineren Volumenunterschieden in der Kammer aufgrund des geringeren Steigrohrradius führen würde. Die Befüllung der Kammer sollte dann über einen separaten Befüllungskanal mit einem kleinen Durchmesser in die Kammer erfolgen, da eine Befüllung über das Steigrohr mit dem kleineren Durchmesser zur Bildung von Lufteinflüssen führen könnte. Der Durchmesser des Befüllungskanals sollte so klein gehalten werden, daß aufgrund der daraus resultierenden grossen Kapillarkräfte das darin befindliche Flüssigkeitsvolumen an dem Entleerungsvorgang nicht teilnimmt. Aus diesem Grunde mündet der Befüllungskanal auch vorteilhafterweise vom Boden her in die Kammer.

Der Siphon sollte in der horizontalen Ebene möglichst dicht neben dem Steigrohr angeordnet sein, um die Lageunempfindlichkeit der Meßvorrichtung zu erhöhen. Die aufgrund einer Neigung der Siphon-Anordnung erfolgende frühere oder spätere Auslösung der Entleerung führt auch hier zu geringeren Unterschieden des Flüssigkeitsvolumens in der Kammer bzw. im Steigrohr.

Zur Vermeidung von zu hohen Kapillarkräften in der Kammer sollte diese mindestens einen Durchmesser von 4 mm haben. Der Durchmesser des Ablaufs sollte hingegen etwas kleiner sein, da sonst die aufgrund der Kapillarwirkung und der Kohäsionswirkung der Flüssigkeit erfolgende selbsttätige Entleerung der Siphon-Anordnung nicht sicher gewährleistet ist.

Die Siphon-Anordnung besteht vorteilhafterweise aus durchsichtigem Kunststoff, zum Beispiel Polyacryl, um die Ankoppelung der Reflex-Lichtschranke der Detektoreinrichtung zu erleichtern.

In Abhängigkeit von dem Medium der Flüssigkeitsströmung und dem Material, in dem die Siphon-Anordnung ausgebildet ist, können zweckmäßigerweise auch Detektoreinrichtungen auf Infrarotbasis, Ultraschallbasis oder elektrostatischer Wirkungsweise eingesetzt werden. Auch eine Detektoreinrichtung zur Erfassung der Magnetfeldänderung zwischen vorhandenem und nicht vorhandenem Meßvolumen ist möglich. Auch hierbei kann das Grundprinzip einer indirekten Messung, die nicht direkt mit der Flüssigkeitsströmung in Kontakt tritt und einer Impuls- bzw. Sprungantwort beibehalten werden, die letztlich das digital einfache Erfassen der Anzahl der Meßvolumina gestattet.

Die Meßvorrichtung eignet sich insbesondere für Flüssigkeitsmessung im klinischen Bereich und dort vor allem für die Urinmessung. Die Meßvorrichtung erlaubt eine automatische Messung und Überwachung der kontinuierlichen Urinabgabe insbesondere von Intensivpatienten. Da aufgrund des Meßverfahrens ein digitales Signal erhalten wird, lassen sich die erhaltenen Informationen sehr gut für eine zeitliche und/oder statistische Auswertung verwenden.

Die Meßvorrichtung kann bei einer Durchflußbandbreite von 5 ml/h bis 3 l/h mit einer Meßgenauigkeit von etwa 2 % oder kleiner eingesetzt werden.

Obwohl die Messung mittels Volumenstromsensor nach dem Siphonprinzip verschiedensten Parametern der Flüssigkeit und der Strömungsgeometrie der Siphonanordnung unterworfen ist werden sehr genaue Meßergebnisse erhalten, die auch durch Eiweißausflockungen und sonstige Verunreinigungen wie z.B. feinste Blutgerinnsel nicht in nennenswertem Maße beeinflußt werden.

Im Hinblick auf eine vollständige Entleerung der Siphon-Anordnung einschließlich Kammer setzt die Siphon-Anordnung vorzugsweise am untersten Bodenniveau, das vorteilhafterweise noch schräg ablaufend gestaltet ist, an, so daß auch kurzfristige Sedimentabsetzungen bei dem nächsten Entleerungsvorgang mit ausgespült werden können.

Die Siphon-Anordnung läßt sich als Durchflußsensor in der Art eines Einwegartikels herstellen. Es sind keine beweglichen Teile und keine elektrischen Anschlüsse notwendig. Die Meßvorrichtung genügt damit den Anforderungen an Kostengünstigkeit, Funktionssicherheit und Hygiene, die an Geräte im klinischen Anwendungsbereich gestellt werden. Durch die Vorrichtung entfällt die bislang für Urinmessung übliche Ablesung der Urinmenge an einen Urinmeßbeutel. Hierbei besteht die Gefahr von Ablese- und Übertragungsfehlern. Die Erfindung kommt somit der kritischen Personalsituation im Krankenhausbereich entgegen, weil die Pflegekräfte von diesen leicht zu automatisierenden manuellen Tätigkeiten entlastet werden und sich intensiver der Patientenbetreuung widmen können.

Die Meßvorrichtung eignet sich hervorragend zur automatischen Überwachung der Nierenfunktion, zum Beispiel in einer Intensiv-Station. Das detektierte Signal kann zum Beispiel mit einem Vergleichssignal verglichen werden und eine automatische Alarmauslösung veranlassen, wenn eine bestimmte Abgabemenge über eine gewisse Zeit hinweg unter- oder überschritten wird.

Die Erfindung erreicht daher durch kleinstmögliche Portionierung der zu messenden Flüssigkeitsvolumina eine größtmögliche Genauigkeit. Fertigungstechnisch wird durch die einfache Konzeption ohne jegliche Hinterschneidungen ein Massenartikel mit relativ niedrigen Herstellungskosten erreicht.

Obwohl in der Beschreibung primär auf die Urinmengenmessung abgestellt ist, kann die Meßvorrichtung selbstverständlich auch für Blutmessungen, die Durchflußmengenbestimmung von Infusionen, aber auch in anderen Bereichen, z.B. bei der Niederschlagsmengenbestimmung von Regenwasser oder Leckagemessung im Bereich der chemischen oder nuklearen Industrie eingesetzt werden.

Die Erfindung wird nachfolgend beispielsweise anhand der schematischen Zeichnung in den Figuren 5, 6, 7, 8, 11 noch näher beschrieben. Die weiteren Figuren 1, 2, 3, 4, 9, 10 dienen nur zur Erläuterung. Hierbei zeigen:
- Fig. 1: einen Längsschnitt durch eine Siphon-Anordnung mit geringer Varianz des Füllgrades;
- Fig. 2: den Schnitt A-A aus Fig. 1;
- Fig. 3: einen Längsschnitt durch eine Siphon-Anordnung mit von unten erfolgender Füllung der Kammer;
- Fig. 4: den Schnitt A-A aus Fig. 3;
- Fig. 5: einen Längsschnitt durch ein weiteres Beispiel einer Siphon-Anordnung;
- Fig. 6: den Querschnitt A-A aus Fig. 5;
- Fig. 7: einen schematischen Längsschnitt durch ein weiteres Ausführungsbeispiel einer Siphon-Anordnung mit trompetenartig erweitertem Ablaufkanal;
- Fig. 8: einen Längsschnitt durch ein weiteres Beispiel einer Siphon-Anordnung mit einem vom Ablaufkanal in den Bereich vor der Kammer zurückgeführten Rückschlußkanal;
- Fig. 9: ein weiteres Beispiel einer Siphon-Anordnung im Längsschnitt, in dem die Kammer und der Siphon durch einen zwischen Führungszapfen eingelegten Schlauch gebildet sind;
- Fig. 10: eine schematische Draufsicht auf die zwei Hälften der Siphon-Anordnung nach Fig. 9 in der Phase des Zusammenfügens, jedoch ohne die Einlage des Schlauchs und
- Fig. 11: eine schematische Blockdarstellung einer Detektoreinrichtung mit Korrelationseinheiten zur Neigungs- und Durchflußberücksichtigung.

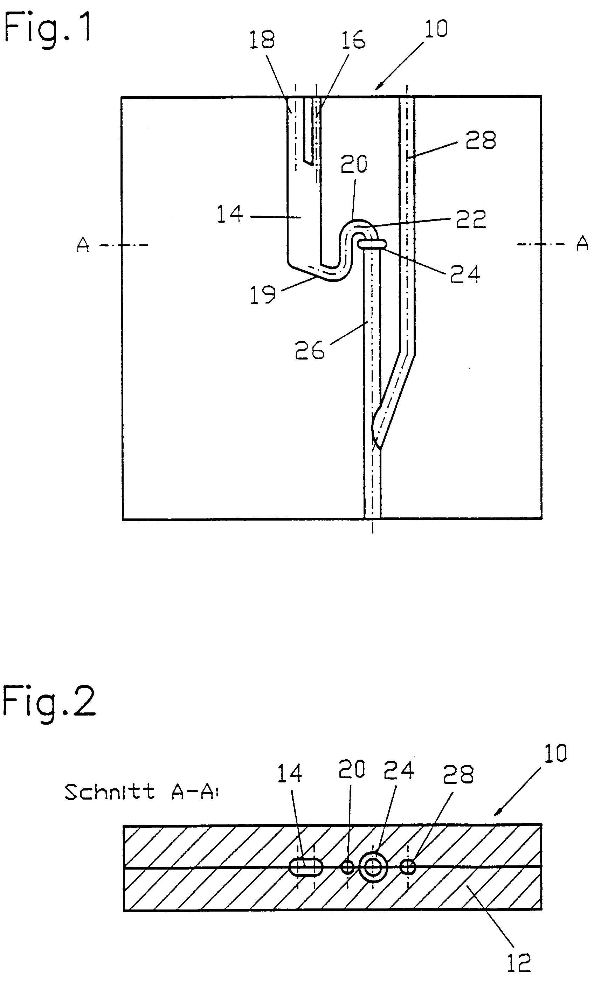

Fig. 1 zeigt eine Siphon-Anordnung 10 in einem Polystyrolblock 12 als Sensor-Anordnung zum Beispiel für die Urinmessung in Krankenhäusern. Diese Siphon-Anordnung 10 besteht zweckmäßigerweise aus zwei zur mittleren Schnittebene spiegelbildlich ausgebildeten Halbblöcken, die z.B. abdichtend zusammengeschraubt werden können, so daß auch relativ komplizierte Konturen der Siphon-Anordnung 10 fertigungstechnisch leicht herstellbar sind. Im Aufstaubereich der Siphon-Anordnung 10 befindet sich eine Kammer 14, in die von oben ein Befüllungskanal 16 und ein Belüftungskanal 18 mündet. Die Kammer 14 geht hierbei über eine zum Siphon 20 hin zumindest leicht abfallende Bodenschräge 19 kontinuierlich in den S- bzw. sinusförmigen Siphon 20 über. Stromabwärts von der oberen Überlaufkante 22 des Siphons 20 hat der Ablauf einen Durchmessersprung 24. Nach diesem Durchmessersprung 24 ist der Ablauf durch einen senkrechten im Vergleich zum Siphon 20 durchmessergrößeren Ablaufkanal 26 gebildet. In den Ablaufkanal 26 mündet in spitzem Winkel ein zweiter Belüftungskanal 28 der mit dem ersten Belüftungskanal 18 für die Kammer 14 oder mit der Umgebungsatmosphäre verbunden sein kann.

Fig. 2 zeigt den Schnitt durch den Polystyrolblock 12 an der Stelle A-A. Der Schnitt dient zur Verdeutlichung der Durchmesserverhältnisse unterschiedlicher Bereiche der Siphon-Anordnung 10.

Die Funktionsweise des Flüssigkeitsstromsensors wird nachfolgend kurz erläutert:

Über den Befüllungskanal 16 wird die Kammer 14 im Aufstaubereich der Siphon-Anordnung 10 entsprechend dem zu messenden Flüssigkeitsstrom mit Flüssigkeit gefüllt, wobei die Flüssigkeit in dem Siphon 20 hochsteigt. Die Flüssigkeit steigt entsprechend der Füllhöhe in der Kammer 14 über die obere Überlaufkante 22 des Siphons 20 und bildet an dem Durchmessersprung 24 eine Flüssigkeitsmembran. Bei weiterem Ansteigen der Flüssigkeit in der Kammer 14 wird bei einer definierten Füllhöhe die Oberflächenspannung der Flüssigkeitsmembran an dem Durchmessersprung 24 überschritten und die gesamte in der Kammer 14 befindliche Flüssigkeit wird durch ihren Kohäsionseffekt durch den Siphon 20 und den Ablaufkanal 26 abruptartig und im wesentlichen vollständig entleert. Diese Entleerung wird durch eine nicht dargestellte Reflex-Lichtschranke vorzugsweise im Bereich der Kammer 14 oder gegebenenfalls im Ablaufkanal gemessen. Hierdurch erhält man ein Signal bei jedem Entleerungszyklus, das aufgrund des definierten Füllvolumens der Meßkammer genauen Aufschluß über die Durchflußrate durch den als Siphon-Anordnung ausgebildeten Sensor 10 ermöglicht.

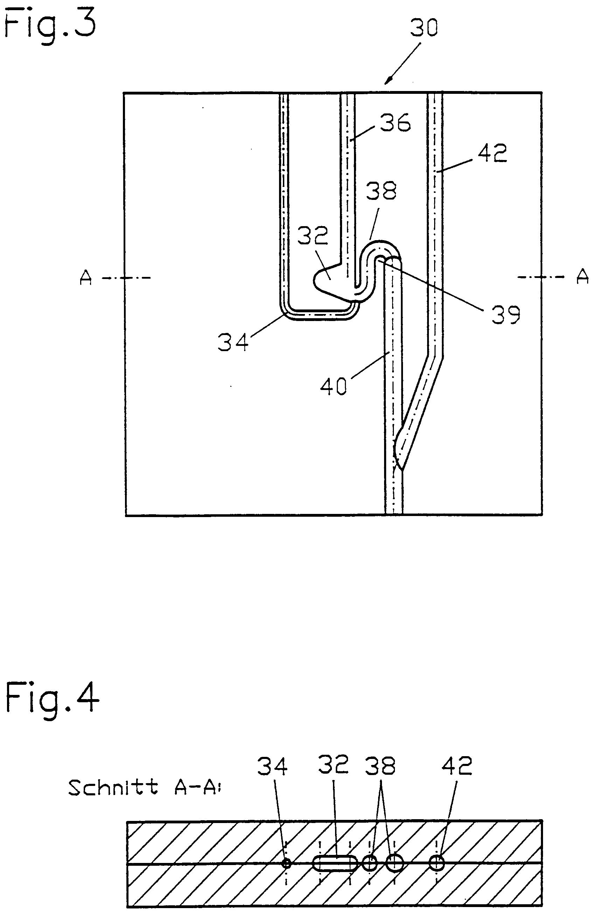

Die Figuren 3 und 4 zeigen eine weitere Siphonanordnung 30. Die Siphon-Anordnung 30 hat eine immer 32, die vom Boden her über einen verglichen mit dem Siphon 38 durchmesserkleinen Befüllungskanal 34 befüllt wird. In die Kammer 32 mündet von oben ein Steigrohr 36 mit einem im Vergleich zur Breite der Kammer 32 geringen Durchmesser. Vom Boden der Kammer 32 führt der siphonförmige Ablauf 38 in einen senkrechten Ablaufkanal 40, in den im spitzen Winkel ein Belüftungskanal 42 mündet, der mit der Atmosphäre oder dem Steigrohr 36 verbunden ist. Der Vorteil dieser Siphon-Anordnung 30 besteht darin, daß das Füllvolumen der Kammer 32 sich bei einer leichten Neigung der Siphon-Anordnung 30 um eine waagerechte Achse nicht sehr stark ändert. Der Entleerungsvorgang wird bei dieser Siphon-Anordnung 30 ausgelöst, wenn die Flüssigkeit die obere Überlaufkante 39 des Siphons 38 übersteigt. Eine Verdrehung der Siphon-Anordnung 30 um eine aus der Zeichenebene herausragende Achse bewirkt eine bezüglich der unverschwenkten Lage frühere oder spätere Auslösung des Entleerungsvorganges, wobei der Flüssigkeitspegel in dem Steigrohr 36 bei Einleitung der Entleerung etwas höher oder niedriger liegt. Dieser Höhenunterschied des Füllstandes bei Entleerung bewirkt jedoch nur eine geringe Änderung des Flüssigkeitsvolumens in der gesamten Kammer 32, da das Steigrohr 36 einen im Vergleich zur Kammer 32 geringen Durchmesser aufweist und somit ein Höhenunterschied des Flüssigkeitsstandes in dem Steigrohr nur zu einer geringen absoluten Volumendifferenz führt. Der geringere Steigrohrdurchmesser 36 macht es notwendig, daß ein eigener Befüllungskanal 34 in die Kammer 32 einmündet. Der Befüllungskanal 34 hat einen verglichen mit dem Ablauf 38 geringen Durchmesser, wodurch das im Befüllungskanal 34 befindliche Flüssigkeitsvolumen nicht am Entleerungsvorgang teilnimmt. Der Siphon 38 ist in horizontaler Ebene sehr dicht am Steigrohr 36 angeordnet, um die Differenz des lageabhängigen Flüssigkeitsstandes im Steigrohr 36 bei Auslösung der Entleerung möglichst gering zu halten.

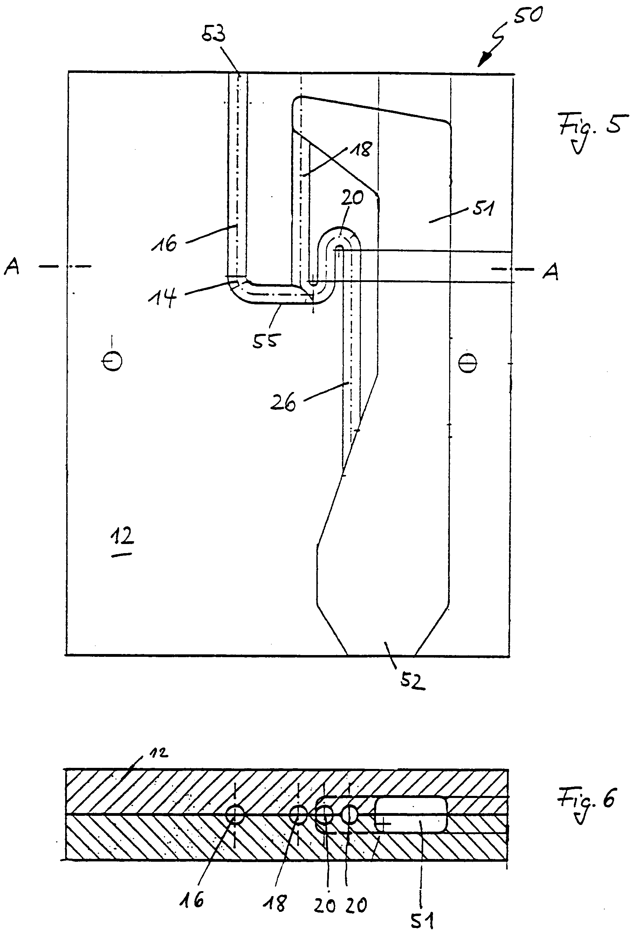

Die Darstellung nach Fig. 5 zeigt ein Ausführungsbeispiel der Erfindung einer Siphon-Anordnung 50 in einer Draufsicht auf die Seitenfläche eines Halbschalenblockes 12 aus durchsichtigem Kunststoff.

Der Siphon 20, der S- bzw. sinus- oder U-förmig ausgebildet ist, geht einerseits in einen vertikal angeordneten Ablaufkanal 26 über. Zulaufseitig weist die Siphonanordnung 50 eine einzige Zulauföffnung 53 auf, die im Beispiel an der oberen Kante des Blockes 12 endet. Von dieser Zulauföffnung 53 führt senkrecht nach unten der Befüllungskanal 16, dessen unterer Teil Teil der Kammer 14 ist. Im Beispiel nach Fig. 5 wird die Kammer 14 daher durch einen kreisförmigen oder ovalen Kanal gebildet, der etwa gleichen Durchmesser wie der Kanal des Siphon 20 aufweist. Geometrisch gesehen wird die Kammer 14 durch einen, etwa waagrecht verlaufenden Kanal gebildet, der im linken Bereich kontinuierlich gekrümmt in den Befüllungskanal 16 und im rechten Bereich ebenfalls kontinuierlich gekrümmt in den aufsteigenden Zweig des Siphon 20 übergeht.

Nahezu parallel und dicht benachbart zum aufsteigenden Zweig des Siphons 20 ist ein Belüftungskanal 18 etwa senkrecht von oben her kommend mit der Kammer 14 im Bereich des Übergangs zum Siphon 20 kommunizierend verbunden. Dieser Belüftungskanal 18 geht in einen wesentlich breiteren und damit mit wesentlich größerem Luftvolumen ausgestatteten Rückflußkanal 51 über, der im oberen Bereich das oberste Niveau des Siphons 20 wesentlich überragt. Dieser Rückflußkanal 51 weist an seiner schmalsten Stelle etwa die dreifache Breite des Kanals der Kammer 14 bzw. des Siphons 20 auf. Der Ablaufkanal 26 mündet im Beispiel nach Fig. 5 in einer Schrägfläche in einen unteren Erweiterungsbereich des Rückflußkanals 51, der an der unteren Kante des Blocks 12 eine Ablauföffnung 52 z.B. zum Anschluß eines Urinsammelbeutels aufweist.

In der Schnittdarstellung gemäß Fig. 6 längs der Schnittlinie A-A nach Fig. 5 ist die Größenabmessung des Rückflußkanals 51 im Vergleich zum Durchmesser des Siphons 20 gut erkennbar. Der Rückflußkanal 51, der als Entlüftungs- und Luftrückleitungskanal dient, weist ein erheblich größeres Volumen auf als Kammer und Siphon-Anordnung zusammen.

Der Vorteil dieser Ausführungsform der Siphonanordnung 50 kann vor allen Dingen darin gesehen werden, daß der Belüftungskanal 18 intern vom Ablaufkanal 26 über den Rückflußkanal 51 zur Kammer zurückgeführt ist. Es besteht daher nur eine Zulauföffnung 53 und eine Ablauföffnung 52, wodurch gerade im Intensivbereich keine Bakterienfilter zur Vermeidung einer Kontamination über den Belüftungskanal benötigt werden. Zudem wird gerade in der Volumenrelation deutlich, daß das Volumen der Kammer und des Siphons 20 auf ein kleinstmögliches Volumen ausgelegt ist, um die unterteilten Meßvolumina der abfließenden Flüssigkeit digital erfassen zu können. Auch bei einem abrupten Abfließen dieser kleinen Meßvolumina ist daher der rasche Druckausgleich durch die Großvolumigkeit im Rückflußkanal 51 gewährleistet. Darüber hinaus wäre auch bei einer relativ starken Blasenentlastung sichergestellt, daß die Kammer 14 und der Siphon 20 ohne Rückstau in den Ablaufkanal 26 hinein impulsartig entleert werden können, da im oberen Bereich des Rückflußkanals 51 ein Auffangvolumen für stoßartig auftretene größere Flüssigkeitsmengen zur Verfügung steht.

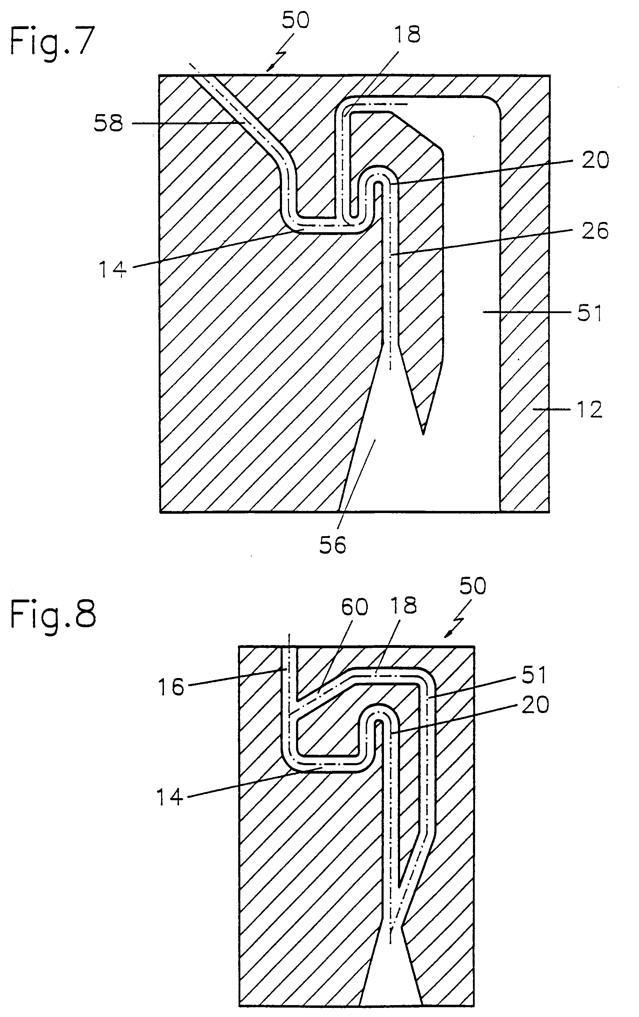

In Fig. 7 ist ein weiteres Ausführungsbeispiel einer etwas abgeänderten Siphonanordnung 50 dargestellt, die jedoch vom Grundprinzip der Kammer und des Siphons 20 mit dem Beispiel nach Fig. 5 vergleichbar ist.

Im Gegensatz z.B. nach Fig. 5 ist der Befüllungskanal 58 in der Fig. 7 als Schrägkanal ausgebildet, der kontinuierlich in den Anfangsbereich der Kammer 14, vorzugsweise oberhalb des obersten Niveaus des Siphons 20, übergeht. Aufgrund dieses Schrägkanals 58 fließt die zu messenden Flüssigkeit entlang der unteren Wand des Schrägkanals 58, so daß Luft im oberen Teil des Schrägkanals entweichen kann und dadurch Lufteinschlüsse, die eine Verfälschung der zu messenden übereinstimmenden Meßvolumina mit sich bringen würden, vermieden werden.

Andererseits ist im Beispiel nach Fig. 7 der senkrecht nach unten geführte Bereich des Ablaufkanals 26 mit einer trompetenförmigen Erweiterung 56 versehen. Durch diese Erweiterung wird eine Tropfenbildung im Auslaßbereich verhindert und die Strömungsdynamik insbesondere im Hinblick auf ein beschleunigtes Abfließen verbessert.

Ein Durchmessersprung 24 (Fig. 1), der eine Verbesserung des Flüssigkeitsmeniskus und damit eine präzisere Auslösungscharakteristik mit sich bringt, kann beispielsweise auch im Einlaßbereich zur Kammer 14, z.B. am Übergang zum Schrägkanal 58 vorhanden sein.

Die schematische Darstellung nach Fig. 8 zeigt ebenfalls die Grundstruktur wie nach den Ausführungsbeispielen der Fig. 5 und 7. Wesentlich ist hier jedoch die Rückführung des Rückflußkanals 51 über den Belüftungskanal 18 mit einer Mündung 60 im Bereich des Einlasses zur Kammer 14.

Diese Ausführungsform zeigt vor allen Dingen bei hohen Durchflußraten der Flüssigkeit, also z.B. bei starker Blasenentleerung, den Vorteil, daß die digital zu erfassenden einzelnen Meßvolumina durch den Einschluß einer Luftblase stets separiert sind und somit auch einzeln erfaßt werden können. Die Rückführung der Belüftung 18 erfolgt hierbei in der Höhe des obersten Siphon-Niveaus, so daß am Ende einer über die Kammer 14 und den Siphon 20 entleerten Flüssigkeitssäule eine Luftblase vorhanden ist. Diese Ausführungsform stellt daher sicher, daß eine geringe Varianz auch bei hohen Durchflußraten erreicht wird, wobei das Prinzip der Lufteinschlüsse mit einer "Perlenschnur" verglichen werden kann.

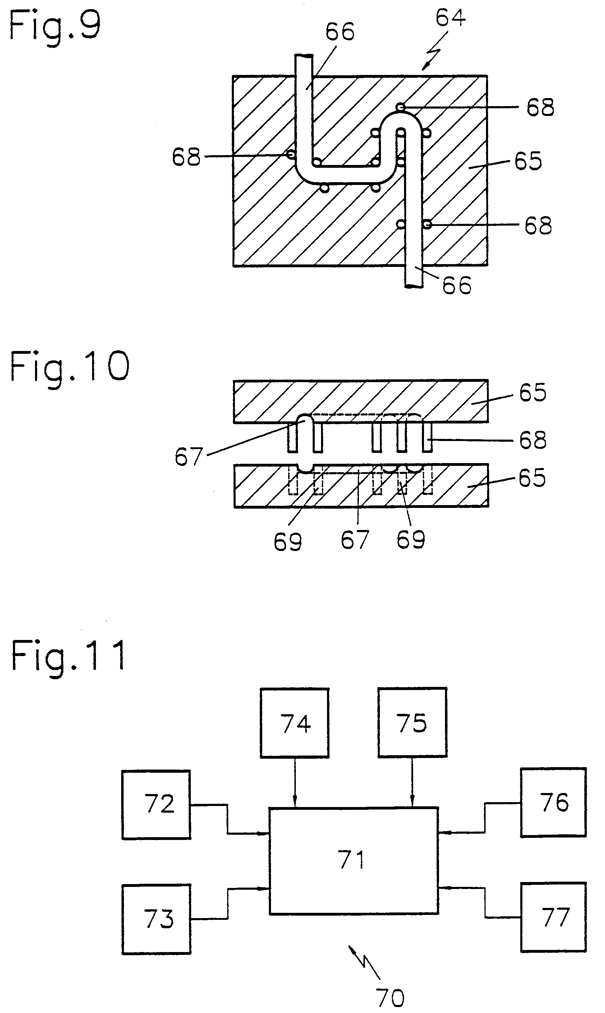

In den Fig. 9 und 10 ist ein weiteres Siphonanordnung schematisch dargestellt. Die eine Ansicht auf die Innenfläche eines Halbblockes 65 darstellende Fig. 9 zeigt, daß die Kanalanordnung aus Befüllungskanal, Kammer, Siphon und Ablaufkanal aus einem nach Möglichkeit durchsichtigen Schlauch 66 gebildet wird, der zwischen Führungszapfen 68 entsprechend der optimierten geometrischen Konfiguration in eine halbkreisförmige Ausnehmung 67 (Fig. 10) paßgenau eingelegt ist.

In Fig. 10 ist in Draufsicht die Zusammenfügbarkeit der beiden weitgehend komplementär ausgebildeten Halbblöcke 65 dargestellt. Die Führungszapfen 68 zur Einlage des Schlauchs 66 dienen gleichzeitig als Verbindungszapfen, die in die komplementären Bohrungen 69 bzw. Vertiefungen paßgenau einfügbar sind. Der Schlauch 66 selbst liegt hierbei in den halbkreisförmigen Ausnehmungen 67 des entsprechenden Halbblockes 65.

Der wesentliche Vorteil dieser Siphonanordnung 64 kann darin gesehen werden, daß hierbei allein der Schlauch 66 im Sinne eines Einwegartikels nach Gebrauch ausgetauscht werden muß. Die Halbblöcke 65 jedoch können unverzüglich oder nach einer Sterilisation wiederverwendet werden, so daß bei diesem Beispiel eine erhebliche Kosteneinsparnis erreicht wird.

Die Darstellung nach Fig. 11 zeigt das elektronische Prinzip der Detektoranordnung 70, in der entsprechende Korrelationseinrichtungen zur Eliminierung von Neigungs- bzw. Durchflußabweichungen enthalten sind. Der Mikroprozessor 71 erhält hierbei einerseits über den Meßstreckenblock 73 Signale für die Anzahl der durchfließenden Meßvolumina, worüber auch die Durchflußgeschwindigkeit erfaßt werden kann. Des weiteren ist ein Block 72 vorgesehen, der als Neigungswinkelgeber die Schrägstellung der Siphonanordnung an den Mikroprozessor 71 weitergibt.

Da die Neigungsanordnung der gesamten Siphonanordnung das Volumen der entsprechenden Flüssigkeit in der Kammer und dem Siphon, wenn auch geringfügig, beeinflußt, ist ein Neigungskorrekturblock 74 vorgesehen, in dem in Art einer Datei bzw. Tabelle die Neigungskompensationsdaten als Funktion des Neigungswinkels abrufbar sind. In vergleichbarer Weise ist auch für unterschiedliche Durchflußgrößen ein Durchflußkorrekturblock 75 mit dem Mikroprozessor 71 verbunden. In diesem tabellen- bzw. dateiartig aufgebauten Korrekturblock 75 sind die Korrekturdaten abrufbar bereitgestellt, die bei unterschiedlichen Durchflußmengen die Meßvolumeneinheit beeinflussen können.

Weiterhin ist schematisch ein Block 76 mit dem Mikroprozessor 71 verbunden, wobei dieser Block 76 Peripheriegeräte, wie Anzeige, Drucker, Alarmgeber, umfassen kann. Auch kann ein Block 77 als Aktuatorblock zur Unterbrechung des Flüssigkeitsflusses mit dem Prozessor 71 verbunden sein. Dieser Aktuator kann dann betätigt werden, wenn z.B. ein Transport oder eine Umbettung eines Intensivpatienten bei angeschlossener Siphonanordnung erfolgt. In diesen Fällen könnte durch eine horizontale Anordnung im Gegensatz zu der betriebsmäßig vertikalen Meßanordnung ein Rückfluß von Flüssigkeit bzw. Urin möglich sein, was jedoch durch den Unterbrechungsaktuator unterbunden wird.

Die Erfindung gestattet daher äußerst kostengünstig eine sehr präzise Messung einer Flüssigkeitsströmung auf digitalisierter Basis, wobei die Kammer und der Siphon auf kleinstmögliche Volumina, insbesondere durch Durchmessergrößen von etwa 4 mm und etwas größer ausgelegt sind.

## Patentansprüche

1. Vorrichtung zur Messung des Gesamtvolumens einer Flüssigkeitsströmung, insbesondere als Urin-Meßvorrichtung, mit einer Siphon-Anordnung, die einen Siphon (20) für eine Unterteilung der Flüssigkeitsströmung in einzelne Teil-Meßvolumina aufweist,
mit einem Befüllungskanal (16), einem Ablaufkanal (26), und einem Belüftungskanal (18) für die Flüssigkeitsströmung, mit einer digitalen Detektoreinrichtung zur Erfassung jedes durch die Siphon-Anordnung initiierten Entleerungszyklus für jedes Teilmeßvolumen, und
mit einer Auswerteinrichtung, die das Gesamtvolumen der Flüssigkeitsströmung aus der Anzahl der Entleerungszyklen und den einzelnen Teilmeßvolumina der über den Siphon ablaufenden Flüssigkeitsströmung ermittelt,
dadurch **gekennzeichnet**,
a) daß in Strömungsrichtung direkt vor der Siphon-Anordnung eine damit kommunizierende etwa waagrecht verlaufende Kammer (14) zur temporären Aufnahme jeweils eines reproduzierbaren Teilmeßvolumens der Flüssigkeitsströmung in der Kammer und den sich anschließenden Siphon (20) vorgesehen ist, wobei die Kammer (14) zwischen dem unteren Bereich des Befüllungskanals (16) und dem aufsteigenden Bereich des Siphons (20) gebildet ist,
b) daß der Belüftungskanal (18, 28; 36, 42) mit einer Luftrückkopplung vom Ablaufkanal (26) direkt zur Kammer (14, 32) oder direkt auf dem Niveau der Überlaufkante des Siphons (20) zum Befüllungskanal (16, 60) innerhalb der Vorrichtung zurückgeführt ist,
c) daß die Übergänge vom Befüllungskanal (16) zur Kammer (14) und von der Kammer (14) zum aufsteigenden Bereich des Siphons (20) kontinuierlich ausgebildet sind, und
d) daß die Überlaufkante des Siphons (20) höher als die Kammer (14, 32) angeordnet ist.

2. Vorrichtung nach Anspruch 1,
dadurch **gekennzeichnet,**
daß die Kammer (14, 32) und der Siphon (20) etwa gleichen, lichten Durchmesser aufweisen.

3. Vorrichtung nach Anspruch 2,
dadurch **gekennzeichnet,**
daß die Kammer (14, 32) und der Siphon (20) etwa gleichen Kreisdurchmesser haben.

4. Vorrichtung nach einem der Ansprüche 1 bis 3,
dadurch **gekennzeichnet,**
daß die Kammer (14) und die Siphon-Anordnung (10) mit Befüllungs- (16, 34), Ablauf- (26, 40) und Belüftungskanal (18, 28; 36, 42) in zwei zu einer mittleren Schnittebene spiegelbildlich ausgebildeten und gegeneinander abgedichteten Halbblöcken (12) aus durchsichtigem Kunststoff ausgebildet sind.

5. Vorrichtung nach einem der Ansprüche 1 bis 4,
dadurch **gekennzeichnet,**
daß der Siphon (20, 38) der Siphon-Anordnung (10, 30) einen Durchmesser von etwa 3,5 mm bis 5 mm, insbesondere von 4 mm, aufweist.

6. Vorrichtung nach einem der Ansprüche 1 bis 5,
dadurch **gekennzeichnet,**
daß der verglichen mit dem Ablaufkanal (26) durchmesserkleinere Befüllungskanal (18) in die Kammer (14) mündet und daß mindestens der Siphon (20) im Bereich seiner Überlaufkante einen Durchmessersprung (24) aufweist.

7. Vorrichtung nach einem der Ansprüche 1 bis 6,
dadurch **gekennzeichnet,**
daß der Befüllungskanal (16) mit einer Kanalschräge (58) in die Kammer (14, 32) übergeht, und daß der Ablaufkanal (26) eine, insbesondere trompetenförmige Erweiterung (56) aufweist.

8. Vorrichtung nach einem der Ansprüche 1 bis 7,
dadurch **gekennzeichnet,**
daß die Oberfläche der flüssigkeitsführenden Teile der Siphon-Anordnung (10) und/oder der Kammer (14) aus hydrophobem Material hergestellt und/oder hydrophob behandelt ist.

9. Vorrichtung nach einem der Ansprüche 1 bis 8,
dadurch **gekennzeichnet,**
daß die Siphon-Anordnung aus durchsichtigem Kunststoff, insbesondere einem biologisch abbaubaren Kunststoff, z.B. Biocellat oder aus Polyacryl oder Polystyrol besteht und daß die Detektoranordnung eine Refelex-Lichtschranke aufweist.

10. Vorrichtung nach einem der Ansprüche 1 bis 9,
dadurch **gekennzeichnet,**
daß die Kammer (14) und die Siphon-Anordnung (10) mit Befüllungs- und Ablaufkanal durch einen zwischen zwei zu einer mittleren Schnittebene im wesentlichen spiegelbildlich ausgebildeten und gegeneinander abgedichteten Halbblöcken (12) eingelegten, austauschbaren Schlauch (66) gebildet sind.

11. Vorrichtung nach einem der Ansprüche 1 bis 10,
dadurch **gekennzeichnet,**
daß die Detektoreinrichtung mindestens einen in den Bereich der Kammer (14) und/oder des Ablaufbereichs (26) der Siphon-Anordnung (10) gerichteten Sensor aufweist.

12. Vorrichtung nach Anspruch 11,
dadurch **gekennzeichnet,**
daß der Sensor als optischer Sensor, Infrarot-Sensor, Ultraschall-Sensor, Magnetfeld-Sensor oder elektrostatischer Sensor ausgelegt ist.

13. Vorrichtung nach einem der Ansprüche 1 bis 12,
dadurch **gekennzeichnet,**
daß eine Korrektureinrichtung (70, 71, 72, 73, 74, 75) zur Berücksichtigung von Neigungsabweichungen und Abweichungen in der Durchflußgeschwindigkeit der einzelnen Meßvolumina vorgesehen ist.

## Claims

1. Apparatus for measuring the total volume of a liquid flow, particularly as a urine measuring apparatus, having a siphon unit, which has a siphon (20) for subdividing the liquid flow into individual partial measuring volumes, having a filling duct (16), an outflow duct (26) and an aerating duct (18) for the liquid flow, having a digital detector means for detecting each emptying cycle for each partial measuring volume initiated by the siphon unit and having an evaluating means, which determines the total volumes of the liquid flow from the number of emptying cycles and the individual partial measuring volumes of the liquid flow taking place via the siphon, characterized in that,
a) in the flow direction directly upstream of the siphon unit is provided a roughly horizontally directed chamber (14) communicating therewith for temporarily receiving in each case a reproducible partial measuring volume of the liquid flow in the chamber and the following siphon (20), the chamber (14) being formed between the lower area of the filling duct (16) and the rising area of the siphon (20),
b) the aerating duct (18,28;36,42) having an air feed-back from the outflow duct (26) directly to the chamber (14,32) or directly to the level of the overflow edge of the siphon (20) to the filling duct (16,60) within the apparatus,
c) the transitions from the filling duct (16) to the chamber (14) and from the chamber (14) to the rising area of the siphon (20) are constructed in continuous manner and
d) the overflow edge of the siphon (20) is positioned higher than the chambers (14,32).

2. Apparatus according to claim 1, characterized in that the chambers (14,32) and the siphon (20) have roughly the same internal diameter.

3. Apparatus according to claim 2, characterized in that the chambers (14,32) and the siphon (20) have roughly the same diameter.

4. Apparatus according to one of the claims 1 to 3, characterized in that the chamber (14) and the siphon unit (10) with the filling (16,34), outflow (26,40) and aerating (18,28;36,42) ducts are constructed in the form of two transparent plastic half-blocks (12) sealed against one another and constructed homologously with respect to a central sectional plane.

5. Apparatus according to one of the claims 1 to 4, characterized in that the siphon (20,38) of the siphon unit (10,30) has a diameter of approximately 3.5 to 5 mm, more particularly 4 mm.

6. Apparatus according to one of the claims 1 to 5, characterized in that the filling duct (18) having a smaller diameter than the outflow duct (26) issues into the chamber (14) and that at least the siphon (20) has a diameter jump (24) in the vicinity of its overflow edge.

7. Apparatus according to one of the claims 1 to 6, characterized in that the filling duct (16) passes with a bevel (58) into the chambers (14,32) and that the outflow duct (26) more particularly has a trumpet-shaped widening (56).

8. Apparatus according to one of the claims 1 to 7, characterized in that the surface of the liquid-carrying parts of the siphon unit (10) and/or the chamber (14) is made from hydrophobic material and/or is hydrophobically treated.

9. Apparatus according to one of the claims 1 to 8, characterized in that the siphon unit is made from transparent plastic, in particular a biodegradable plastic, e.g. Biocellat or a polyacrylic or polystyrene material and that the detector unit has a reflected light barrier.

10. Apparatus according to one of the claims 1 to 9, characterized in that the chamber (14) and the siphon unit (10) with filling and outflow ducts are formed by an interchangeable hose (66) inserted between two half-blocks (12) sealed against one another and constructed substantially homologously with respect to a central sectional plane.

11. Apparatus according to one of the claims 1 to 10, characterized in that the detector means has at least one sensor directed into the vicinity of the chamber (14) and/or the outflow area (26) of the siphon unit (10).

12. Apparatus according to claim 11, characterized in that the sensor is designed as an optical sensor, infrared sensor, ultrasonic sensor, magnetic field sensor or electrostatic sensor.

13. Apparatus according to one of the claims 1 to 12, characterized in that a correcting device (70,71,72,73,74,75) is provided for taking account of inclination variations and divergences in the flow rate of the individual measuring volumes.

## Revendications

1. Dispositif pour la mesure du volume total d'un flux de liquide, en particulier un dispositif de mesure d'urine, comprenant :
- un dispositif à siphon, équipé d'un siphon (20) servant à diviser le flux liquide en volumes élémentaires de mesure individuels,
- un canal de remplissage (16), un canal de sortie (26) et un canal d'aération (18) du flux liquide,
- un dispositif numérique de détection pour détecter chaque cycle de vidange déclenché par le dispositif à siphon pour évacuer chacun des volumes élémentaires de mesure,
- un dispositif d'exploitation donnant le volume total de flux de liquide, à partir du nombre des cycles de vidange et des volumes élémentaires de mesure individuels du flux liquide évacués individuellement à travers le siphon, dispositif caractérisé en ce que :
a) il est prévu, directement en amont du dispositif à siphon, par rapport au sens de l'écoulement, une chambre sensiblement horizontale (14), communiquant avec le siphon, pour accueillir temporairement à chaque fois un volume élémentaire de mesure reproductible du flux de liquide dans la chambre et dans le siphon qui lui est raccordé, la chambre (14) étant formée entre la partie inférieure du canal de remplissage (16) et la partie ascendante du siphon (20),
b) le canal d'aération (18, 28 ; 36, 42) est ramené au canal de remplissage (16, 60) à l'intérieur du dispositif par une réinjection d'air allant du canal de sortie (26) directement à la chambre (14, 32) ou directement au niveau du bord de déversement du siphon (20),
c) les passages du canal de remplissage (16) à la chambre (14) et de celle-ci à la partie ascendante du siphon (20) ont un tracé continu,
d) le bord de déversement du siphon (20) est situé plus haut que la chambre (14, 32).

2. Dispositif selon la revendication 1, caractérisé en ce que la chambre (14, 32) et le siphon (20) ont à peu près la même section de passage.

3. Dispositif selon la revendication 2, caractérisé en ce que la chambre (14, 32) et le siphon (20) ont à peu près le même diamètre circulaire.

4. Dispositif selon une des revendications 1 à 3, caractérisé en ce que la chambre (14) et le dispositif à siphon (10) comprenant les canaux de remplissage (16, 34) de sortie (26, 40) et d'aération (18, 28 ; 36, 42) sont formés dans deux demi-blocs (12) d'un plastique transparent, symétriques par rapport à un plan de coupe médian et étanches l'un par rapport à l'autre.

5. Dispositif selon une des revendications 1 à 4, caractérisé en ce que le siphon (20, 38) du dispositif (10, 30) a un diamètre compris entre 3,5 et 5 mm, en particulier de 4 mm.

6. Dispositif selon une des revendications 1 à 5, caractérisé en ce que le canal d'aération (18), de diamètre inférieur à celui du canal de sortie (26), débouche dans la chambre (14) tandis qu'au moins le siphon (20), au niveau de son bord de déversement, présente une variation brusque de diamètre (24).

7. Dispositif selon une des revendications 1 à 6, caractérisé en ce que le canal d'aération (16) se prolonge vers la chambre (14, 22) par une partie en pente (58) , tandis que le canal de sortie (26) présente un élargissement (56), en forme de trompette notamment.

8. Dispositif selon une des revendications 1 à 7, caractérisé en ce que la surface des parties du dispositif à siphon (10) et/ou de la chambre (14) sur laquelle circule le flux liquide est faite d'un matériau hydrophobe et/ou est traitée de manière hydrophobe.

9. Dispositif selon une des revendications 1 à 6, caractérisé en ce que le dispositif à siphon est fait d'une matière plastique transparente, en particulier une matière plastique éliminable par voie biologique, par exemple du biocellat ou de la résine polyacrylique ou du polystyrène, le dispositif de détection comportant une barrière lumineuse à réflexion.

10. Dispositif selon une des revendications 1 à 9, caractérisé en ce que la chambre (14) et le dispositif à siphon (10) avec ses canaux d'aération et de sortie sont constitués d'un tube (66) amovible monté entre deux demi-blocs (12) essentiellement symétriques par rapport à un plan de coupe médian et étanches l'un par rapport à l'autre.

11. Dispositif selon une des revendications 1 à 10, caractérisé en ce que le dispositif de détection comporte au moins un détecteur opérant au niveau de la chambre (14) et/ou de la sortie (26) du dispositif à siphon (10).

12. Dispositif selon la revendication 11, caractérisé en ce que le détecteur est un détecteur optique, ou à infrarouge, ou à ultrasons, ou à champ magnétique ou électrostatique.

13. Dispositif selon une des revendications 1 à 12, caractérisé en ce qu'un dispositif de correction (70, 71, 72, 73, 74, 75) est prévu pour prendre en compte les variations d'inclinaison et les écarts que présentent les vitesses de passage des divers volumes élémentaires de mesure.
